# EUROPÄISCHE PATENTSCHRIFT

(11) **EP 4 195 926 B1**
(45) Veröffentlichungstag und Bekanntmachung des Hinweises auf die Patenterteilung: **21.08.2024**
(21) Anmeldenummer: 21732519.0
(22) Anmeldetag: 18.05.2021
(51) Int. Cl.: A01N 31/02, A01N 25/16, A01P 1/00

(54) **ALKOHOLHALTIGER DESINFEKTIONSSCHAUM**
ALCOHOL-BASED DISINFECTANT FOAM
MOUSSE DÉSINFECTANTE À BASE D'ALCOOL

(30) Priorität: 14.08.2020 DE 102020004965
(43) Veröffentlichungstag der Anmeldung: 21.06.2023
(73) Patentinhaber: nsc pharma GmbH & Co. KG, 48268 Greven (DE)
(72) Erfinder: DANIELS, Rolf, 72108 Rottenburg (DE)
(74) Vertreter: Gulde & Partner
(86) Internationale Anmeldenummer: PCT/IB2021/054269
(87) Internationale Veröffentlichungsnummer: WO 2022/034386

(56) Entgegenhaltungen:
- WO-A1-2011/039630
- WO-A2-2008/155389
- US-A- 5 167 950
- US-A1- 2010 189 809
- US-A1- 2012 214 878
- .: "Phospholipon 90G", 1 March 2007 (2007-03-01), Köln, Germany, pages 1 - 1, XP055833180, Retrieved from the Internet <URL:http://www.americanlecithin.com/TDS/TDS_90G.PDF> [retrieved on 20210819]
- DATABASE GNPD [online] MINTEL; 21 October 2020 (2020-10-21), ANONYMOUS: "Nourishing Hand Sanitizer", XP055833127, retrieved from https://www.gnpd.com/sinatra/recordpage/8204417/ Database accession no. 8204417

## Beschreibung

Die vorliegende Erfindung betrifft einen Desinfektionsschaum auf der Basis von Alkoholen zur Desinfektion der Haut. Der erfindungsgemäße Desinfektionsschaum vermeidet die bei alkoholischen Desinfektionsmitteln übliche Störung der Hautbarriere und kommt ohne Silikontenside und ohne polyfluorierte Chemikalien aus.

Die Desinfektion der Haut, insbesondere der Hände, ist für viele medizinische Zwecke unumgänglich. Spätestens seit dem Auftreten der Covid-19-Pandemie ist auch eine regelmäßige Desinfektion der Hände im Haushalt notwendig geworden. Für die Desinfektion haben sich Alkohole, insbesondere Ethanol, bewährt. Die Verwendung von flüssigen Alkoholen zur Desinfektion der Haut hat jedoch einige Nachteile: Einerseits neigen desinfizierende Alkohole dazu, die Hautbarriere zu stören, insbesondere die Haut auszutrocknen. Insbesondere in den Wintermonaten kommt es bei regelmäßiger Anwendung zu trockener Haut, da ab Temperaturen von unter 8 Grad Celsius die körpereigene Talgproduktion eingestellt wird. Problematisch ist aber auch die gleichmäßige Verteilung auf der Hand. Darüber hinaus neigt flüssiger Alkohol dazu, von der Hand abzutropfen. Flüssiger Alkohol auf dem Boden stellt eine Rutschgefahr dar, das Auftropfen von Alkohol auf Kleidung oder Schuhe führt zu Fleckenbildung.

Aus anderen kosmetischen Anwendungen hat sich die Darreichungsform eines Schaums bewährt, da Schäume gut dosierbar sind und sich gleichmäßig auf der Haut verteilen lassen. Es hat bereits Versuche gegeben, alkoholhaltige Schäume als Desinfektionsmittel zu etablieren. Auf Grund der verminderten Grenzflächenspannung von Alkohol und Luft im Vergleich zu Wasser stellt sich die Herstellung eines stabilen Schaumes als Herausforderung dar.

EP 1858323 B1 beschreibt z. B. einen alkoholhaltigen Schaum, der mittels silikonhaltiger Tenside hergestellt wurde. Derartige silikonhaltige Tenside stellen allerdings ein Umweltproblem dar, da sie nur sehr wenig bioabbaubar sind.

WO 00/47183 A beschreibt alkoholhaltige Gele zur Hautdesinfektion, wobei die Gele durch silikonhaltige Polymere und durch polyfluorierte Chemikalien (z.B. Perfluorpolypropylether) stabilisiert sind. Auch polyfluorierte Chemikalien sind ökologisch bedenklich.

US 9023374 B2 (basierend auf WO 2011/039630 A1) beschreibt alkoholhaltige Zusammensetzungen zur Desinfektion, enthaltend Docusat-Natrium (Natriumdioctylsulfosuccinat). Die Zusammensetzungen sind allerdings nicht zur Ausbildung stabiler Schäume geeignet (siehe Beispiele, unten).

US 2016/0250111 A1 beschreibt Handdesinfektionsmittel auf alkoholischer Basis, jedoch keine Schäume.

US 5,167,950 offenbart alkoholische antimikrobielle Schäume auf der Basis von Acrylsäurepolymeren und ethoxylierten Tensiden.

US 2010/0189809 alkoholische antimikrobielle Schäume auf der Basis von polyfluorierten Tensiden.

Es besteht daher Bedarf an verbesserten Desinfektionsschäumen auf der Basis von Alkohol, die ohne Silikontenside und ohne polyfluorierte Chemikalien auskommen.

Überraschenderweise wurde gefunden, dass derartige Desinfektionsschäume ohne Verwendung von Silikontensiden und ohne polyfluorierte Chemikalien hergestellt werden können. Die erfindungsgemäßen Formulierungen enthalten hydrierte Phospholipide zur Schaumstabilisierung, insbesondere Phospholipon 80H und/oder Phospholipon 90H.

Die hydrierten Phospholipide werden bevorzugt aus Phospholipiden natürlichen Ursprungs gewonnen. Insbesondere eignen sich Phospholipide aus Pflanzen, wie beispielsweise Sojabohnenlecithin. Charakterisiert werden können die Phospholipide durch den Phosphatidylcholingehalt, bei dem es sich um den Hauptinhaltsstoff von Phospholipiden handelt. Aus diesen nativen Phospholipiden werden die erfindungsgemäß eingesetzten hydrierten Phospholipide durch Hydrierung hergestellt. Besonders günstig erweisen sich solche Phospholipide die aufgereinigt sind und einen hohen Anteil an Phosphatidylcholin aufweisen.

Bei den hydrierten Phospholipiden beträgt der Phosphatidylcholingehalt mindestens etwa 70 Gew.-%.

Das erfindungsgemäß verwendete hydrierte Phospholipid kann auch eine Mischung verschiedener hydrierter Phospholipide und insbesondere eine Mischung aus hydriertem Phosphatidylcholin und hydriertem Lysophosphatidylcholin sein. In einer solchen Mischung sollte das Gewichtsverhältnis von hydriertem Phosphatidylcholin zu hydriertem Lysophosphatidylcholin zwischen 97:3 und 40:60 betragen.

Bekannte Phospholipide, die diese Eigenschaften erfüllen, sind beispielsweise von der Phospholipid GmbH (Köln) unter den Bezeichnungen Phospholipon 80H und Phospholipon 90 H erhältlich. Phospholipon 80H umfaßt etwa 76% hydriertes Phosphatidylcholin und etwa 3% hydriertes Lysophosphatidylcholin, Phospholipon 90 H, ein hydriertes Phosphatidylcholin, umfaßt mindestens 90% hydriertes Phosphatidylcholin und maximal 4% hydriertes Lysophosphatidylcholin. Hydrierte Phospholipide anderer Hersteller können jedoch auch für die erfindungsgemäßen Zubereitungen verwendet werden.

Der Anteil der hydrierten Phospholipide in der aufschäumbaren Zubereitung beträgt 0,5 bis 5 Gew.%. Auch eine Mischung verschiedener hydrierter Phospholipide ist geeignet. Diese hydrierten Phospholipide werden bei einer Temperatur zwischen 40 und 60 °C unter Rühren zu einer wässrigen Alkohollösung gegeben.

Als Alkohole sind insbesondere Ethanol, n-Propanol und Isopropanol geeignet, wovon Ethanol besonders bevorzugt ist. Der Alkoholgehalt der Lösung beträgt mindestens 50 Gew%, alkoholische Lösungen von 60 bis 75 Gew% sind besonders bevorzugt. Auch Mischungen der genannten Alkohole sind geeignet.

Die Erfindung betrifft daher schäumbare alkoholhaltige Zusammensetzungen zur Desinfektion der Haut, die frei von Silikontensiden und frei von polyfluorierten Chemikalien sind, enthaltend
a) Ethanol, n-Propanol und/oder i-Propanol in einer wässrigen Lösung mit einem Alkoholgehalt von mehr als 50%

und b) ein oder mehrere hydrierte Phospholipide in einem Anteil von 0,5-5% bezogen auf die Gesamtmenge der Flüssigkeit in Gewichtsprozent,
gemäß Anspruch 1.

Weitere vorteilhafte Ausgestaltungen sind Gegenstand der Unteransprüche.

Die beschriebene Zubereitung kann weiter stabilisiert werden durch einen Anteil an Ethanol-löslichen Celluloseethern, wie z. B. Tylose PSO, typischerweise weniger als 1 Gew%.

Ferner kann die Zubereitung bis zu 10 Gew% Glycerol und bis 6 Gew% Wasserstoffperoxid enthalten.

Als besonders vorteilhaft hat sich ein Zusatz von 1-10 Gew% (bevorzugt 2,5-7,5 Gew%) Harnstoff (Urea) herausgestellt: Erfindungsgemäße Schäume behalten auch mit Zusatz von Harnstoff ihre Schaumstruktur und die vorteilhaften Desinfektionseigenschaften. Gleichzeitig verringern harnstoffhaltige Schäume gelegentlich auftretende Hautempfindlichkeiten gegen die Alkohole.

Eine Reihe erfindungsgemäßer Zusammensetzungen sind in den Tabellen 1 bis 12 beispielhaft angeführt. Die in den Tabellen 1 bis 12 gezeigten Zusammensetzungen werden zunächst gemischt (typischerweise durch Einrühren der hydrierten Phospholipide bei 40 bis 60 °C zu der wässrigen Alkohol¬lösung) und dann in geeignete Druckbehältnisse aus Glas, Aluminium, Weißblech oder Kunststoff abgefüllt, mit einem Schaumventil, z. B. Precision-Ventil, verschlossen und danach bevorzugt mit ca. 2 bis 10 Gew% eines Gases oder einer Gasmischung (z. B. Propan/Butan) beaufschlagt. Durch das Gas oder die Gasmischung wird bei Raumtemperatur ein Gleichgewichtsdruck von 1-5 bar erreicht. Durch Betätigung des Sprühkopfes können die gewünschten Mengen an Schaum über einen handelsüblichen Schaumkopf, z.B. Modell "Fiji" von Precision Dispensing Solutions Europe, entnommen werden.

Die erfindungsgemäßen Produkte sind dazu geeignet, die Haut, insbesondere die Hand wirksam zu desinfizieren. Hierzu wird eine ca. walnussgroße Menge Schaum aus einem Spender entnommen und gleichmäßig auf der gewünschten Körperstelle, insbesondere der Hand, verteilt. Der Schaum zeichnet sich durch eine hervorragende Haftung auf der Haut aus. Durch die Haftung wird auch das Abtropfen von Alkohol verhindert. Es bildet sich ein Schutzschirm auf der Haut, der in Wasser schwer löslich ist, und als Schutzbarriere wirkt. Die Desinfektion kann regelmäßig erfolgen, ohne Haut auszutrocknen oder anderweitig zu reizen. Der erfindungsgemäße Schaum ist bakterizid, viruzid, levurozid und myobakterizid.

Die erfindungsgemäßen Zusammensetzungen können die aus ähnlichen Schäumen bekannten Emulgatoren und Emulsionsstabilisatoren, Pflegemittel, Viskositätsregler, Tenside sowie pH-Puffer enthalten. Auch die Zugabe von filmbildenden Polymeren ist möglich. Zu nennen sind hier insbesondere Polyvinyllalkohol, PVP, Copovidone, Celluloseether, Polyvinyl-co-Polymere, und Polymethacrylat-co-Polymere. Die Summe dieser optionalen Zusätze sollte 5% nicht überschreiten.

Ein Zusatz von Duftstoffen ist möglich, wird aber zur Vermeidung von Allergierisiken nicht empfohlen.

Beispielhaft werden einige Zusammensetzungen in den nachfolgenden

Tabellen offenbart, ohne dass diese eine Beschränkung darstellen sollen. Der Fachmann auf dem Gebiet kann zahlreiche Variationen und Weiterentwicklungen vornehmen, ohne erfinderisch tätig werden zu müssen.

Sofern nicht anders angegeben sind alle %-Angaben in dieser Schrift Gewichtsprozente (Gew%).

### Beispiele

**Tabelle 1:**

| **Inhaltsstoff** | **A** | **B** | **C** | **D** | **E** | **F** |
|---|---|---|---|---|---|---|
| **Phospholipon 80H** | 0,5 - 5 % | | | 0,5 - 5 % | | |
| **Phospholipon 90 H** | | 0,5 - 5 % | | | 0,5 - 5 % | |
| **Andere hydrierte Phospholipide** | | | 0,5 - 5 % | | | 0,5 - 5 % |
| **Ethanol 70%** | Ad 100% | Ad 100% | Ad 100% | | | |
| **Ethanol 60%** | | | | Ad 100% | Ad 100% | Ad 100% |

| | | | | | | |
|---|---|---|---|---|---|---|
| Beaufschlagt mit 2 - 10 % Propan-Butan-Gemisch mit einem Gleichgewichtsdruck von 1 - 5 bar (das heißt: zu 100g der Lösungen mit den o.g. Zusammensetzungen werden jeweils 2 bis 10g Propan-Butan-Gemisch gegeben). | | | | | | |

**Tabelle 2:**

| **Inhaltsstoff** | **A** | **B** | **C** | **D** | **E** | **F** |
|---|---|---|---|---|---|---|
| **Phospholipon 80H** | 0,5 - 5 % | | | 0,5 - 5 % | | |
| **Phospholipon 90 H** | | 0,5 - 5 % | | | 0,5 - 5 % | |
| **Andere hydrierte Phospholipide** | | | 0,5 - 5 % | | | 0,5 - 5 % |
| **Glycerol** | 5 % | 5 % | 5 % | 5 % | 5 % | 5 % |
| **Ethanol 70%** | Ad 100% | Ad 100% | Ad 100% | | | |
| **Ethanol 60%** | | | | Ad 100% | Ad 100% | Ad 100% |

| | | | | | | |
|---|---|---|---|---|---|---|
| Beaufschlagt mit 2 - 10 % Propan-Butan-Gemisch mit einem Gleichgewichtsdruck von 1 - 5 bar | | | | | | |

**Tabelle 3:**

| **Inhaltsstoff** | **A** | **B** | **C** | **D** | **E** | **F** |
|---|---|---|---|---|---|---|
| **Phospholipon 80H** | 0,5 - 5 % | | | 0,5 - 5 % | | |
| **Phospholipon 90 H** | | 0,5 - 5 % | | | 0,5 - 5 % | |
| **Andere hydrierte Phospholipide** | | | 0,5 - 5 % | | | 0,5 - 5 % |
| **Tylose PSO** | 0,5 % | 0,5 % | 0,5 % | 0,5 % | 0,5 % | 0,5 % |
| **Ethanol 70%** | Ad 100% | Ad 100% | Ad 100% | | | |
| **Ethanol 60%** | | | | Ad 100% | Ad 100% | Ad 100% |

| | | | | | | |
|---|---|---|---|---|---|---|
| Beaufschlagt mit 2 - 10 % Propan-Butan-Gemisch mit einem Gleichgewichtsdruck von 1 - 5 bar | | | | | | |

**Tabelle 4:**

| **Inhaltsstoff** | **A** | **B** | **C** | **D** | **E** | **F** |
|---|---|---|---|---|---|---|
| **Phospholipon 80H** | 0,5 - 5 % | | | 0,5 - 5 % | | |
| **Phospholipon 90 H** | | 0,5 - 5 % | | | 0,5 - 5 % | |
| **Andere hydrierte Phospholipide** | | | 0,5 - 5 % | | | 0,5 - 5 % |
| **Glycerol** | 5 % | 5 % | 5 % | 5 % | 5 % | 5 % |
| **Tylose PSO** | 0,5 % | 0,5 % | 0,5 % | 0,5 % | 0,5 % | 0,5 % |
| **Ethanol 70%** | Ad 100% | Ad 100% | Ad 100% | Ad 100% | Ad 100% | Ad 100% |

| | | | | | | |
|---|---|---|---|---|---|---|
| Beaufschlagt mit 2 - 10 % Propan-Butan-Gemisch mit einem Gleichgewichtsdruck von 1 - 5 bar | | | | | | |

**Tabelle 5:**

| **Inhaltsstoff** | **A** | **B** | **C** | **D** | **E** | **F** |
|---|---|---|---|---|---|---|
| **Phospholipon 80H** | 0,5 - 5 % | | | 0,5 - 5 % | | |
| **Phospholipon 90 H** | | 0,5 - 5 % | | | 0,5 - 5 % | |
| **Andere hydrierte Phospholipide** | | | 0,5 - 5 % | | | 0,5 - 5 % |
| **i-Propanol 70%** | Ad 100% | Ad 100% | Ad 100% | | | |
| **i-Propanol 60%** | | | | Ad 100% | Ad 100% | Ad 100% |

| | | | | | | |
|---|---|---|---|---|---|---|
| Beaufschlagt mit 2 - 10 % Propan-Butan-Gemisch mit einem Gleichgewichtsdruck von 1 - 5 bar | | | | | | |

**Tabelle 6:**

| **Inhaltsstoff** | **A** | **B** | **C** | **D** | **E** | **F** |
|---|---|---|---|---|---|---|
| **Phospholipon 80H** | 0,5 - 5 % | | | 0,5 - 5 % | | |
| **Phospholipon 90 H** | | 0,5 - 5 % | | | 0,5 - 5 % | |
| **Andere hydrierte Phospholipide** | | | 0,5 - 5 % | | | 0,5 - 5 % |
| **n-Propanol 70%** | Ad 100% | Ad 100% | Ad 100% | | | |
| **n-Propanol 60%** | | | | Ad 100% | Ad 100% | Ad 100% |

| | | | | | | |
|---|---|---|---|---|---|---|
| Beaufschlagt mit 2 - 10 % Propan-Butan-Gemisch mit einem Gleichgewichtsdruck von 1 - 5 bar | | | | | | |

**Tabelle 7:**

| **Inhaltsstoff** | **A** | **B** | **C** | **D** | **E** | **F** |
|---|---|---|---|---|---|---|
| **Phospholipon 80H** | 0,5-2,5% | | 0,5-2,5% | 0,5 - 5 % | | 0,5-2,5% |
| **Phospholipon 90 H** | 0,5-2,5% | 0,5 - 5 % | | 0,5-2,5% | 0,5 - 5 % | |
| **Andere hydrierte Phospholipide** | | 0,5-2,5% | 0,5 - 5 % | | 0,5-2,5% | 0,5 - 5 % |
| **Ethanol 70%** | Ad 100% | Ad 100% | Ad 100% | | | |
| **i-Propanol 60%** | | | | Ad 100% | Ad 100% | Ad 100% |

| | | | | | | |
|---|---|---|---|---|---|---|
| Beaufschlagt mit 2 - 10 % Propan-Butan-Gemisch mit einem Gleichgewichtsdruck von 1 - 5 bar | | | | | | |

**Tabelle 9:**

| **Inhaltsstoff** | **A** | **B** | **C** | **D** | **E** | **F** |
|---|---|---|---|---|---|---|
| **Phospholipon 80H** | 0,5 - 5 % | | | 0,5 - 5 % | | |
| **Phospholipon 90 H** | | 0,5 - 5 % | | | 0,5 - 5 % | |
| **Andere hydrierte Phospholipide** | | | 0,5 - 5 % | | | 0,5 - 5 % |
| **Glycerol** | 6 % | 8 % | 10 % | 6 % | 8 % | 10 % |
| **Ethanol 80%** | Ad 100% | Ad 100% | Ad 100% | | | |
| **Ethanol 75%** | | | | Ad 100% | Ad 100% | Ad 100% |

| | | | | | | |
|---|---|---|---|---|---|---|
| Beaufschlagt mit 2 - 10 % Propan-Butan-Gemisch mit einem Gleichgewichtsdruck von 1 - 5 bar | | | | | | |

**Tabelle 10:**

| **Inhaltsstoff** | **A** | **B** | **C** | **D** | **E** | **F** |
|---|---|---|---|---|---|---|
| **Phospholipon 80H** | 0,5 - 5 % | | | 0,5 - 5 % | | |
| **Phospholipon 90 H** | | 0,5 - 5 % | | | 0,5 - 5 % | |
| **Andere hydrierte Phospholipide** | | | 0,5 - 5 % | | | 0,5 - 5 % |
| **Glycerol** | 10 % | 8 % | 6 % | 10 % | 8 % | 6 % |
| **Tylose PSO** | 0,5 % | 0,5 % | 0,5 % | 0,5 % | 0,5 % | 0,5 % |
| **Ethanol 75%** | Ad 100% | Ad 100% | Ad 100% | Ad 100% | Ad 100% | Ad 100% |

| | | | | | | |
|---|---|---|---|---|---|---|
| Beaufschlagt mit 2 - 10 % Propan-Butan-Gemisch mit einem Gleichgewichtsdruck von 1 - 5 bar | | | | | | |

**Tabelle 11:**

| **Inhaltsstoff** | **A** | **B** | **C** | **D** | **E** | **F** |
|---|---|---|---|---|---|---|
| **Phospholipon 80H** | 0,5 - 5 % | | | 0,5 - 5 % | | |
| **Phospholipon 90 H** | | 0,5 - 5 % | | | 0,5 - 5 % | |
| **Andere hydrierte Phospholipide** | | | 0,5 - 5 % | | | 0,5 - 5 % |
| **Glycerol** | 10 % | 8 % | 6 % | 10 % | 8 % | 6 % |
| **Harnstoff** | 2,5 % | 5 % | 7,5 % | 7,5 % | 5 % | 2,5 % |
| **Ethanol 75%** | Ad 100% | Ad 100% | Ad 100% | Ad 100% | Ad 100% | Ad 100% |

| | | | | | | |
|---|---|---|---|---|---|---|
| Beaufschlagt mit 2 - 10 % Propan-Butan-Gemisch mit einem Gleichgewichtsdruck von 1 - 5 bar | | | | | | |

**Tabelle 12:**

| **Inhaltsstoff** | **A** | **B** | **C** | **D** | **E** | **F** |
|---|---|---|---|---|---|---|
| **Phospholipon 80H** | 0,5 - 5 % | | | 0,5 - 5 % | | |
| **Phospholipon 90 H** | | 0,5 - 5 % | | | 0,5 - 5 % | |
| **Andere hydrierte Phospholipide** | | | 0,5 - 5 % | | | 0,5 - 5 % |
| **Harnstoff** | 2,5 % | 5 % | 7,5 % | 7,5 % | 5 % | 2,5 % |
| **Ethanol 80%** | Ad 100% | Ad 100% | Ad 100% | Ad 100% | Ad 100% | Ad 100% |

| | | | | | | |
|---|---|---|---|---|---|---|
| Beaufschlagt mit 2 - 10 % Propan-Butan-Gemisch mit einem Gleichgewichtsdruck von 1 - 5 bar | | | | | | |

### Vergleichsbeispiel

**Tabelle 13**

| **Inhaltsstoff** | **A** | **B** | **C** | **D** | **E** | **F** |
|---|---|---|---|---|---|---|
| **Phospholipon 90 H** | 6,0 | 5,0 | | | 6,0 | 6,0 |
| **Phosphatidylcholin** | | | 6,0 | 6,0 | | |
| **Glycerol** | | 1,0 | | 2,0 | | 2,0 |
| **Docusat-Natrium** | | | | 0,2 | 0,2 | 0,2 |
| **Menthol** | | | | 0,2 | | |
| **Geraniol** | | | | 0,2 | | |
| **Vitamin E acetat** | | | | 0,6 | | |
| **Ethanol** | 65,0 | 65,0 | 65,0 | 65,0 | 65,0 | 65,0 |
| **Wasser** | Ad 100% | Ad 100% | Ad 100% | Ad 100% | Ad 100% | Ad 100% |

Die Zusammensetzungen gemäß der Tabelle 13 (A-F) werden jeweils unter Rühren bei 40-60°C gemischt, in einen Druckbehälter eingefüllt und mit ca 5 % Propan-Butan-Gemisch beaufschlagt. Der Druckbehälter wird mit einem handelsüblichen Sprühkopf für Schäume versehen. Die Zusammensetzung aus Tabelle 13C entspricht dem Beispiel 34 der US 9,023,374 B2.

Bei Raumtemperatur wird versucht, mittels Betätigung des Schaumkopfes, eine ca. walnussgroße Menge eines Schaumes zu entnehmen.

**Ergebnis:** Die Zusammensetzungen gemäß Tabelle 13A und B ergeben exzellente, feinporige Schäume. Der entnommene Schaum bleibt mehrere Minuten stabil. Der Schaum lässt sich ausgezeichnet auf der Haut verteilen, ohne zu tropfen.

Die Zusammensetzungen gemäß Tabelle 13C bis D ergeben sehr grobporige Schäume, die bereits bei der Entnahme einen gewissen Flüssigkeitsanteil aufweisen. Der Schaumanteil zerfließt unmittelbar nach der Entnahme. Bereits nach deutlich weniger als einer Minute ist praktisch kein Schaum mehr vorhanden. Bei einem Versuch, den Schaum auf der Hand zu verteilen, tropft Flüssigkeit von der Hand ab.

## Patentansprüche

1. Schäumbare alkoholhaltige Zusammensetzung zur Desinfektion der Haut, die frei von Silikontensiden und frei von polyfluorierten Chemikalien sind, enthaltend
a) Ethanol, n-Propanol und/oder i-Propanol in einer wäßrigen Lösung mit einem Alkoholgehalt von mehr als 50 Gew%
und b) ein oder mehrere hydrierte Phospholipide in einem Anteil von 0,5-5 Gew% bezogen auf die Gesamtmenge der Flüssigkeit.

2. Schäumbare alkoholhaltige Zusammensetzung zur Desinfektion der Haut gemäß Anspruch 1, wobei die ein oder mehrere hydrierte Phospholipide ausgewählt sind aus
einer Mischung von etwa 76% hydriertem Phosphatidylcholin und etwa 3% hydriertem Lysophosphatidylcholin
und/oder
einer Mischung von mindestens 90% hydriertem Phosphatidylcholin und maximal 4% hydriertem Lysophosphatidylcholin.

3. Schäumbare alkoholhaltige Zusammensetzung zur Desinfektion der Haut gemäß Anspruch 1, wobei die wässrige Lösung von Ethanol, n-Propanol und/oder i-Propanol einen Alkoholgehalt von 60-75 Gew% aufweist.

4. Schäumbare alkoholhaltige Zusammensetzung zur Desinfektion der Haut gemäß Anspruch 1, wobei der Alkohol Ethanol ist.

5. Schäumbare alkoholhaltige Zusammensetzung zur Desinfektion der Haut gemäß mindestens einem der Ansprüche 1-4, enthaltend bis zu 10 Gew% Glycerol.

6. Schäumbare alkoholhaltige Zusammensetzung zur Desinfektion der Haut gemäß mindestens einem der Ansprüche 1-5, enthaltend bis zu 1 Gew% Celluloseether.

7. Schäumbare alkoholhaltige Zusammensetzung zur Desinfektion der Haut gemäß mindestens einem der Ansprüche 1-6, optional enthaltend 1 bis 10 Gew% Harnstoff.

8. Druckbehälter mit einem Sprühkopf für Schäume, enthaltend eine schäumbare alkoholhaltige Zusammensetzung zur Desinfektion der Haut gemäß mindestens einem der Ansprüche 1-7, beaufschlagt mit 2-10 Gew% eines Gases oder einer Gasmischung mit einem Gleichgewichtsdruck von 1-5 bar.

9. Alkoholhaltiger Desinfektionsschaum zur Desinfektion der Haut, dadurch erhältlich, dass eine schäumbare alkoholhaltige Zusammensetzung gemäß mindestens einem der Ansprüche 1-7, beaufschlagt mit 2-10 Gew% eines Gases oder einer Gasmischung mit einem Gleichgewichtsdruck von 1-5 bar aus einem Druckbehälter mit einem Sprühkopf für Schäume entnommen wird.

## Claims

1. A foamable, alcohol-based composition for disinfecting skin, which is free of silicone surfactants and free of polyfluorinated chemicals, containing:
a) ethanol, n-propanol and/or i-propanol in an aqueous solution with an alcohol content of more than 50 wt%
and b) one or more hydrogenated phospholipids in a proportion of 0.5-5 wt% in relation to the total amount of the liquid.

2. The foamable, alcohol-based composition for disinfecting skin according to Claim 1, wherein the one or more hydrogenated phospholipids are selected from
a mixture of approximately 76 % hydrogenated phosphatidylcholine and approximately 3 % hydrogenated lysophosphatidylcholine
and/or
a mixture of at least 90 % hydrogenated phosphatidylcholine and at most 4 % hydrogenated lysophosphatidylcholine.

3. The foamable, alcohol-based composition for disinfecting skin according to Claim 1, wherein the aqueous solution of ethanol, n-propanol and/or i-propanol has an alcohol content of 60-75 wt%.

4. The foamable, alcohol-based composition for disinfecting skin according to Claim 1, wherein the alcohol is ethanol.

5. The foamable, alcohol-based composition for disinfecting skin according to at least one of Claims 1-4, containing up to 10 wt% glycerol.

6. The foamable, alcohol-based composition for disinfecting skin according to at least one of Claims 1-5, containing up to 1 wt% cellulose ether.

7. The foamable, alcohol-based composition for disinfecting skin according to at least one of Claims 1-6, optionally containing 1 to 10 wt% urea.

8. A pressure container having a spray nozzle for foams, containing a foamable, alcohol-based composition for disinfecting skin according to at least one of Claims 1-7, subjected to 2-10 wt% of a gas or a gas mixture having an equilibrium pressure of 1-5 bar.

9. An alcohol-based disinfectant foam for disinfecting skin, obtainable by withdrawing a foamable, alcohol-based composition according to at least one of Claims 1-7, subjected to 2-10 wt% of a gas or a gas mixture having an equilibrium pressure of 1-5 bar, from a pressure container having a spray nozzle for foams.

## Revendications

1. Composition moussante contenant de l'alcool pour la désinfection de la peau, exempte de tensioactifs siliconés et exempte de produits chimiques polyfluorés, contenant
a) de l'éthanol, du n-propanol et/ou de l'i-propanol dans une solution aqueuse ayant une teneur en alcool supérieure à 50 % en poids
et b) un ou plusieurs phospholipides hydrogénés dans une proportion de 0,5 à 5 % en poids par rapport à la quantité totale de liquide.

2. Composition moussante contenant de l'alcool pour la désinfection de la peau selon la revendication 1, dans laquelle les un ou plusieurs phospholipides hydrogénés sont choisis parmi
un mélange d'environ 76 % de phosphatidylcholine hydrogénée et d'environ 3 % de lysophosphatidylcholine hydrogénée
et/ou
un mélange d'au moins 90 % de phosphatidylcholine hydrogénée et d'un maximum de 4 % de lysophosphatidylcholine hydrogénée.

3. Composition moussante contenant de l'alcool pour la désinfection de la peau selon la revendication 1, dans laquelle la solution aqueuse d'éthanol, de n-propanol et/ou d'i-propanol présente une teneur en alcool de 60 à 75 % en poids.

4. Composition moussante contenant de l'alcool pour la désinfection de la peau selon la revendication 1, dans laquelle l'alcool est l'éthanol.

5. Composition moussante contenant de l'alcool pour la désinfection de la peau selon au moins l'une quelconque des revendications 1 à 4, contenant jusqu'à 10 % en poids de glycérol.

6. Composition moussante contenant de l'alcool pour la désinfection de la peau selon au moins l'une quelconque des revendications 1 à 5, contenant jusqu'à 1 % en poids d'éther de cellulose.

7. Composition moussante contenant de l'alcool pour la désinfection de la peau selon au moins l'une quelconque des revendications 1 à 6, contenant éventuellement de 1 à 10 % en poids d'urée.

8. Récipient sous pression avec une tête de pulvérisation pour mousses, contenant une composition moussante contenant de l'alcool pour la désinfection de la peau selon au moins l'une quelconque des revendications 1 à 7, chargée avec 2 à 10 % en poids d'un gaz ou d'un mélange de gaz ayant une pression d'équilibre de 1 à 5 bars.

9. Mousse désinfectante contenant de l'alcool pour désinfecter la peau, pouvant être obtenue en prélevant une composition moussante contenant de l'alcool selon au moins l'une quelconque des revendications 1 à 7, chargée avec 2 à 10 % en poids d'un gaz ou d'un mélange de gaz ayant une pression d'équilibre de 1 à 5 bars, à partir d'un récipient sous pression avec une tête de pulvérisation pour mousses.
